# FASCICULE DE BREVET EUROPEEN

(11) **EP 3 110 464 B1**
(45) Date de publication et mention de la délivrance du brevet: **01.11.2017**
(21) Numéro de dépôt: 15705658.1
(22) Date de dépôt: 26.01.2015
(51) Int. Cl.: A61L 27/16, A61L 27/50

(54) **COPOLYMERE ACRYLIQUE, HYDROPHOBE, RETICULE, A BASE DE 2-PHENOXY-TETRAETHYLENE-GLYCOL ACRYLATE, POUR LENTILLES INTRAOCULAIRES**
VERNETZTES HYDROPHOBES ACRYLSÄURECOPOLYMER AUS 2-PHENOXY-TETRAETHYLEN-GLYCOL-ACRYLAT FÜR INTRAOKULARLINSEN
CROSSLINKED, HYDROPHOBIC ACRYLIC COPOLYMER MADE OF 2-PHENOXY-TETRAETHYLENE-GLYCOL ACRYLATE AND INTENDED FOR INTRAOCULAR LENSES

(30) Priorité: 27.02.2014 FR 1451578
(43) Date de publication de la demande: 04.01.2017
(73) Titulaire: Acrylian, 67100 Strasbourg (FR)
(72) Inventeur: TERRISSE, Jean, F-67100 Strasbourg (FR)
(74) Mandataire: Merckling, Norbert
(86) Numéro de dépôt international: PCT/FR2015/050172
(87) Numéro de publication internationale: WO 2015/128555

(56) Documents cités:
- FR-A1- 2 930 731
- US-A1- 2009 088 493

## Description

La présente invention concerne un nouveau matériau polymère acrylique, hydrophobe, parfaitement adapté pour la réalisation de lentilles intraoculaires.

L'invention concerne également des lentilles intraoculaires réalisées à partir d'un tel matériau polymère.

Les lentilles intraoculaires sont des implants ou prothèses ophtalmologiques qui sont mises en place chirurgicalement dans l'oeil de patients souffrant par exemple de cataracte, en remplacement de leur cristallin défaillant.

Lors de cette intervention chirurgicale, le chirurgien pratique une petite incision dans la cornée du patient à travers laquelle il retire le cristallin naturel malade du patient. Puis, il met en place à travers cette incision la lentille intraoculaire dans le sac cristallinien à la place du cristallin retiré.

Cette lentille comporte classiquement une partie optique correctrice, dont la correction varie selon les cas de 10 à 30 dioptries. Cette partie optique est sensiblement en forme de disque et présente une section généralement biconvexe asymétrique. Elle doit être positionnée perpendiculairement et de manière centrée par rapport à l'axe optique de l'oeil.

De cette partie optique centrale s'étendent des prolongements latéraux appelés haptiques, dont le rôle est de tendre les parois du sac cristallinien et d'assurer un positionnement correct de la lentille par rapport à celles-ci.

Afin que l'intervention soit la moins traumatisante possible pour le patient et pour éviter le développement d'un astigmatisme postopératoire, l'incision réalisée dans la cornée doit être la plus petite possible.

Lors de l'intervention chirurgicale de pose de la lentille intraoculaire, la lentille est roulée sur elle-même dans un injecteur dont l'extrémité de sortie est introduite à travers l'incision jusqu'au sac cristallinien.

La lentille intraoculaire, qui présente un diamètre nettement supérieur à la longueur de l'incision, doit être fortement comprimée pour pouvoir être expulsée à travers l'extrémité de sortie de l'injecteur dont le diamètre extérieur est inférieur à celui de l'incision.

Une fois libérée dans le sac cristallinien, la lentille intraoculaire doit se déployer rapidement pour pouvoir se positionner correctement et être capable de remplir sa fonction de correction optique de manière satisfaisante.

En raison de leur nature les destinant à être implantées à demeure à l'intérieur d'un oeil humain, de la fonction optique qu'elles doivent accomplir et de leur procédé de pose très contraignant, les lentilles intraoculaires sont soumises à de très nombreuses contraintes et doivent remplir simultanément de nombreux critères pour être jugées satisfaisantes.

D'un point de vue optique, les lentilles intraoculaires doivent être réalisées dans un matériau transparent d'indice optique suffisant, c'est-à-dire supérieur à 1,5, capable de focaliser sur la macula une fois la lentille en place, tout en ayant un encombrement minimal.

Ce matériau doit permettre de réaliser un usinage de grande précision pour obtenir la qualité optique nécessaire.

D'autre part, les lentilles ne doivent pas causer de problème d'éblouissement et ne doivent pas blanchir ni devenir diffusives avec le temps ou lors de changements de température dans la gamme des températures pouvant être usuellement rencontrées.

Le matériau utilisé pour leur réalisation doit être compatible avec une implantation permanente dans l'oeil humain et ne doit pas être cytotoxique. Il ne doit pas, au fil du temps, diffuser de produits toxiques pour ne pas provoquer de nécroses.

En outre, pour que la lentille puisse être posée sans problème, le matériau doit être suffisamment souple pour pouvoir être plié et enroulé sur lui-même. Il doit résister à une élongation importante et à la pression de poussée sans se rompre, ni casser le tube d'injection, de manière à passer par un orifice d'éjection de diamètre extrêmement réduit, de l'ordre de 1,5 mm ou même moins.

Enfin, une fois dans l'oeil du patient, la lentille intraoculaire doit être capable de se déployer toute seule en quelques secondes, sans rester collée sur elle-même, afin de se positionner correctement dans le sac cristallinien et de retrouver ses caractéristiques optiques.

De nombreuses lentilles intraoculaires, de forme et de composition variées, ont été proposées dans l'art antérieur. Cependant, malgré la très grande diversité proposée, aucune n'a réussi jusqu'à présent à remplir l'ensemble de ces critères de façon satisfaisante.

Le but de l'invention est de fournir un nouveau matériau permettant la réalisation de lentilles intraoculaires remplissant l'ensemble de ces conditions.

Dans l'art antérieur, on a tenté de développer des matériaux plus déformables pour réaliser des lentilles intraoculaires plus faciles à introduire à travers une incision de plus en plus petite.

Bien que plus déformables, les lentilles en matières plastiques dites « hydrophiles » posent des problèmes d'inflammation de l'oeil, du fait de la diffusion de produits s'échappant de ces lentilles difficilement purifiables et toujours en équilibre avec l'eau de l'oeil dans lequel elles sont implantées.

En outre, les matériaux hydrophiles, tels que les hydrogels classiquement utilisés pour réaliser des lentilles intraoculaires accélèrent la migration des cellules épithéliales sur la surface des lentilles et peuvent ainsi être responsable à long terme d'une opacification capsulaire particulièrement gênante pour le patient.

On s'est alors tourné vers les matières plastiques dites « hydrophobes » qui sont définies par une reprise en eau inférieure à 5% à 35°C et qui présentent des caractéristiques propres qui ne dépendent pas de la quantité d'eau absorbée. Lors de la fabrication, elles peuvent être facilement purifiées et débarrassées des produits extractibles, insolubles dans l'eau.

Il s'agit par exemple de polymères acryliques ou à base de silicone.

La souplesse de ces matériaux dépend de la température à laquelle ils se trouvent. Ils présentent une température de transition vitreuse (Tg) en dessous de laquelle ils sont durs et peuvent être usinés et au-dessus de laquelle ils deviennent souples, déformables et élastiques.

Pour la réalisation de lentilles intraoculaires, on doit choisir un matériau présentant une température de transition vitreuse suffisamment basse pour que la lentille résultante soit assez souple pour être roulée et étirée à la température d'une salle d'opération, soit environ 18 à 20°C.

L'invention se place dans le cadre de ces matières plastiques dites « hydrophobes » et visent plus spécifiquement les polymères acryliques.

Le problème bien connu de ces matériaux hydrophobes est que plus ils sont souples et déformables et plus ils deviennent collants.

De ce fait, les lentilles intraoculaires peuvent avoir du mal à se déployer correctement lorsqu'elles sont implantées dans l'oeil du patient. En particulier, les haptiques restent très souvent collées à la partie optique de la lentille.

Pour résoudre ce problème technique, un matériau polymère acrylique destiné à la réalisation de lentilles intraoculaires a été proposé dans la demande de brevet antérieure FR 2 930 731.

Ce matériau est obtenu par polymérisation radicalaire à partir d'un mélange qui comprend les monomères suivants :
- un arylalcoxy-acrylate ou un arylalcoxy-méthacrylate ;
- un alkylacrylate, de préférence l'acrylate de butyle ;
- un acrylate hydroxylé ;
- un méthacrylate hydroxylé ;
- un diacrylate de diol ; et
- un diméthacrylate de diol.

La déformabilité du polymère résultant a été considérablement améliorée par l'ajout dans le mélange initial de monomères, d'un agent de transfert tel que le butane thiol ou l'octane thiol.

Mais même si ce matériau polymère possède des qualités indéniables par rapport aux autres matériaux disponibles sur le marché, il présente deux inconvénients majeurs qui l'empêchent de résoudre le problème technique de façon satisfaisante.

Premièrement, ce matériau reste collant en surface ce qui empêche le déploiement rapide de la lentille intraoculaire une fois celle-ci libérée dans le sac cristallinien.

Avec un tel matériau polymère, il arrive encore que les haptiques restent collées à la partie optique de la lentille, contraignant le chirurgien à tenter de les décoller manuellement à travers l'incision de la cornée. Cette opération risquée est particulièrement délicate.

En outre, les lentilles réalisées avec ce matériau polymère présentent une certaine sensibilité au blanchiment dans l'eau tiède, également appelée « glistening » qui s'avère problématique.

Le phénomène de « glistening » est un effet indésirable qui affecte fréquemment et principalement les lentilles acryliques hydrophobes en modifiant la transparence de leur optique. Lorsque ces lentilles sont immergées dans un milieu aqueux, comme c'est le cas lorsqu'elles sont implantées dans l'oeil d'un patient, il se forme des micro-vacuoles d'eau au sein du matériau polymère qui sont visibles du fait de la différence d'indice optique existant entre l'eau et le polymère.

Comme la quantité d'eau qui est absorbée par le matériau est variable en fonction de la température, la formation de vacuoles est influencée par les variations de température. Lorsque la température varie brusquement, par exemple lorsque le patient venant de l'extérieur entre, en hiver dans un lieu chauffé, ou en été dans un lieu climatisé, ou inversement, des vacuoles d'eau apparaissent ou disparaissent ce qui modifie localement la transparence de la lentille intraoculaire et provoque un blanchiment localisé. On constate localement l'apparition de turbidités, de scintillements ou de « nuages » blanchâtres dans le champ de vision qui peuvent provoquer une gêne et une diminution de l'acuité visuelle.

L'invention offre une solution différente à ce problème en fournissant pour la réalisation de lentilles intraoculaires, un nouveau matériau non collant par nature, et qui présente une très faible sensibilité au blanchiment, en particulier nettement inférieure à celle du matériau antérieur évoqué ci-dessus.

De façon surprenante, ce nouveau matériau résout à la fois le problème du glistening et celui du décollement des haptiques et du déploiement rapide de la lentille lorsqu'elle est implantée dans l'oeil, tout en conservant les caractéristiques indispensables pour l'application visée et les avantages du matériau antérieur précédemment cité.

Pour résoudre ce problème technique, l'invention fournit un nouveau matériau polymère acrylique, hydrophobe, destiné à la réalisation de lentilles intraoculaires.

Ce matériau est un copolymère réticulé d'au moins les monomères suivants :
- un arylalcoxy-acrylate différent d'un 2-phénoxy-(2-éthoxy)ₙ-acrylate avec 4 ≤ n ≤ 6 ;
- un 2-phénoxy-(2-éthoxy)ₙ-acrylate avec 4 ≤ n ≤ 6, de préférence le 2-phénoxy-2-éthoxy-2-éthoxy-2-éthoxy-2-éthoxy-acrylate ;
- un acrylate hydroxylé ;
- un méthacrylate hydroxylé ;
- un diacrylate de diol éthoxylé ; et
- un diméthacrylate de diol éthoxylé.

Ce copolymère réticulé se présente sous la forme d'un réseau macromoléculaire tridimensionnel à chaînes pendantes, du fait de la présence d'au moins un agent de transfert dans le mélange de monomères lors de la réticulation.

Le polymère ne contient plus de monomères d'alkylacrylate et en particulière d'acrylate de butyl, car les présents inventeurs ont observé qu'il était particulièrement responsable du caractère collant des surfaces.

En revanche, il contient le 2-phénoxy-(2-éthoxy)ₙ-acrylate (avec 4 ≤ n ≤ 6) comme monomère. Grâce à ses fonctions éthoxy qui se retrouvent uniformément réparties dans le polymère résultant, ce co-monomère augmente légèrement le caractère amphiphile du matériau. La solubilité de l'eau dans la matière est légèrement augmentée mais reste contrôlée.

La présence de sous-chaînes éthoxylées permet toutefois de répartir en de nombreux sous-domaines de petite taille à l'échelle du réseau macromoléculaire, les surconcentrations d'eau pouvant se manifester lors de petites variations de température. Ces petits sous-domaines sont répartis de manière homogène et sont fixés au réseau. Ils ne peuvent donc pas se regrouper pour capter une plus grande quantité d'eau.

Il ne se forme plus de vacuoles d'eau de tailles suffisantes pour être visibles en lumière naturelle et être ainsi responsables du phénomène de glistening. Celles-ci sont remplacées, par des clusters d'eau plus nombreux mais beaucoup plus petits (de taille nanométrique), localisés au niveau des sous-chaines polyethoxylées courtes, qui du fait de leur très faible taille ne sont pas diffusif de la lumière naturelle.

En outre, ces clusters sont localisés sur des sous-domaines de plus faible indice (1,48 environ) que celui du matériau environnant (autour de 1.54). Le contraste avec l'indice de l'eau (1,33) est plus faible, ce qui rend ces clusters moins visibles.

Pour toutes ces raisons, la sensibilité de la matière au blanchiment (glistening) est fortement diminuée.

Ce monomère permet également de considérablement réduire le caractère autocollant des surfaces sur elles-mêmes comme il sera démontré par la suite.

L'invention enseigne également un procédé de fabrication du matériau polymère acrylique selon l'invention, selon lequel :
- on réalise un mélange contenant au moins un arylalcoxy-acrylate différent d'un 2-phénoxy-(2-éthoxy)ₙ-acrylate avec 4 ≤ n ≤ 6, un 2-phénoxy-(2-éthoxy)ₙ-acrylate avec 4 ≤ n ≤ 6, un acrylate hydroxylé, un méthacrylate hydroxylé, un diacrylate de diol éthoxylé, un diméthacrylate de diol éthoxylé, et un agent de transfert ;
- on polymérise ce mélange par voie radicalaire, en une seule étape de polymérisation, de manière à obtenir par cette polymérisation un réseau macromoléculaire tridimensionnel à chaînes pendantes.

Après polymérisation radicalaire, le polymère est débarrassé des fractions résiduelles de monomères et autres additifs n'ayant pas polymérisé.

Après cette étape de purification, on obtient un matériau qui présente les propriétés physiques indiquées dans la présente demande.

L'invention fournit enfin des lentilles intraoculaires à implanter chirurgicalement dans le sac cristallinien d'un patient, en remplacement de son cristallin naturel, réalisées à partir du matériau polymère acrylique selon l'invention.

De telles lentilles intraoculaires sont particulièrement avantageuses, car le matériau polymère selon l'invention remplit tous les critères nécessaires pour surmonter les nombreuses contraintes liées à l'application visée.

En effet, il présente un indice optique important, supérieur à 1,5, mais insuffisant pour provoquer les phénomènes de réflexions multiples causant le problème d'éblouissement. Cet indice est préférentiellement compris entre 1,53 et 1,56, avec une valeur préférentielle de 1,545.

Ce matériau présente une faible température de transition vitreuse, lui permettant d'être particulièrement souple, déformable et élastique à la température d'implantation de la lentille et à la température de l'oeil. Sa température de transition vitreuse est avantageusement inférieure ou égale à 5°C et par exemple de l'ordre de 2 à 3°C.

Il reste malgré cette basse température de transition vitreuse aisément usinable par enlèvement de copeaux à des températures d'usinage de -15 à-20°C. La variation de propriétés physiques est en effet très brutale dès -5°C.

Il présente une grande aptitude à la déformation sans rupture aux températures d'utilisation, c'est-à-dire entre 18 et 35°C. Avec un module d'élasticité inférieur à 0,4 MPa à 30°C et un allongement à la rupture supérieur ou égal à 250% sous compression, il peut être facilement roulé et fortement étiré dans la cartouche d'injection pour être implanté dans l'oeil du patient.

Grâce à sa tension de surface importante liée à la présence des monomères hydroxylés, le matériau selon l'invention est non collant sur lui-même à l'état sec, comme à l'état humide. Il peut donc se déployer facilement et entièrement une fois en position dans l'oeil du patient et résout ainsi de façon satisfaisante le problème des haptiques restant collées à la partie optique des lentilles intraoculaires.

Ce déploiement se fait de manière rapide du fait du faible temps de relaxation du matériau selon l'invention qui est inférieur à 15 secondes à une température de 20°C et inférieur à 5 secondes à 30°C.

Le matériau purifié présente un taux d'absorption d'eau inférieur à 4% à une température de 40°C et inférieur à 3,5% à 30°C.

D'autres caractéristiques et avantages de l'invention apparaîtront à la lecture de la description détaillée qui va suivre, en particulier le rôle, la nature préférentielle et la quantité de chacun des monomères et autres constituants du mélange permettant d'obtenir le matériau selon l'invention.

Pour faciliter la bonne compréhension du lecteur, cette description est accompagnée à titre d'exemple des dessins annexés suivants :
- la figure 1 est un premier exemple de lentille intraoculaire pouvant être réalisée à partir du matériau selon l'invention ;
- la figure 2 est un deuxième exemple de lentille intraoculaire pouvant être réalisée à partir du matériau selon l'invention.

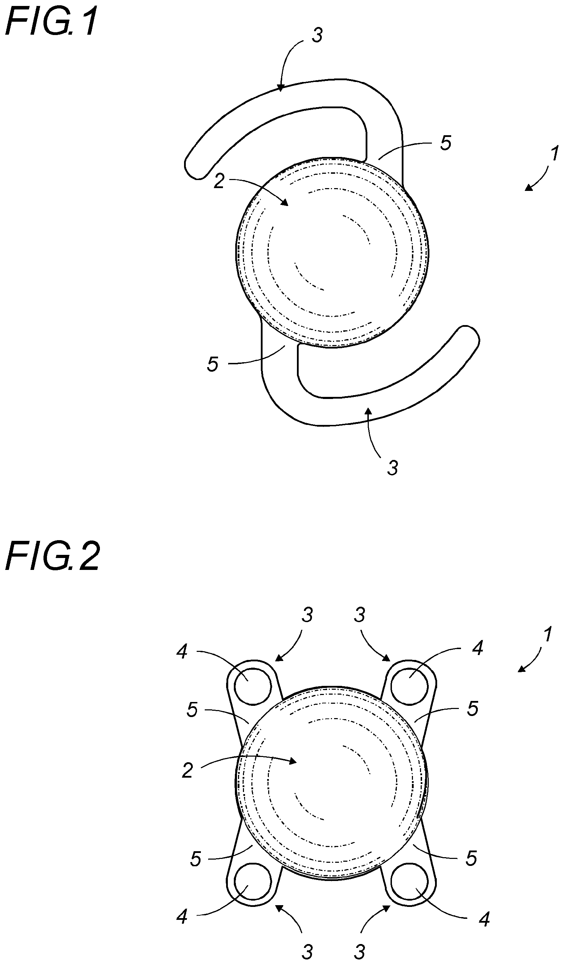

Sur les figures 1 et 2, on a représenté deux exemples classiques de lentille intraoculaire 1 pouvant être réalisée à partir du matériau polymère acrylique selon l'invention.

Ces lentilles 1 comportent une partie optique 2 centrale, sensiblement en forme de disque et à profil bi-convexe.

De cette partie optique 2 s'étendent des prolongements latéraux appelés haptiques 3.

Sur la figure 1, ces haptiques 3 sont au nombre de deux. Elles sont disposées de manière diamétralement opposée et présentent une forme de bras recourbé allant chacun dans un sens opposé.

La lentille de la figure 2 comporte quatre haptiques 3 en forme d'anneau percé d'un orifice central 4. Ces haptiques 3 sont régulièrement réparties sur le pourtour de la partie optique 2.

Sur les exemples représentés, les haptiques 3 sont réalisées d'une pièce avec la partie optique 2 de la lentille 1. Ce type de lentille est appelé « lentille monobloc ». Le matériau selon l'invention est parfaitement adapté à la réalisation de telles lentilles.

Les haptiques 3 sont reliées à la partie optique centrale 2 par une zone de jonction 5 formant charnière qui génère un effet de ressort par rappel élastique de la matière pour déplier la lentille lors de son implantation dans l'oeil d'un patient.

Le matériau selon l'invention est particulièrement adapté à la réalisation de telles lentilles intraoculaires 1.

Il s'agit d'un copolymère acrylique, hydrophobe et réticulé, d'au moins les monomères suivants :
- un arylalcoxy-acrylate différent d'un 2-phénoxy-(2-éthoxy)ₙ-acrylate avec 4≤n≤6 ;
- un 2-phénoxy-(2-éthoxy)ₙ-acrylate avec 4 ≤ n ≤ 6;
- un acrylate hydroxylé ;
- un méthacrylate hydroxylé ;
- un diacrylate de diol éthoxylé ; et
- un diméthacrylate de diol éthoxylé.

Sa structure physique correspond à un réseau macromoléculaire tridimensionnel qui comporte localement des chaînes pendantes. Elle est due à l'action, lors de la réticulation d'un agent de transfert ajouté au mélange de monomères avant polymérisation.

L'utilisation d'un arylalcoxy-acrylate, qui présente une température de transition vitreuse relativement basse, permet d'obtenir un polymère final d'indice optique élevé.

On peut utiliser comme arylalcoxy-acrylate différent d'un 2-phénoxy-(2-éthoxy)ₙ-acrylate avec 4 ≤ n ≤ 6, un composé choisi parmi le 2-phénoxy-éthylacrylate, le 2-phénoxy-2-éthoxy-éthylacrylate ou le 2-phénoxy-2-éthoxy-2-éthoxy-éthylacrylate.

On peut citer à titre d'exemple préférentiel le 2-phénoxy-éthylacrylate.

Le mélange initial avant polymérisation comprend préférentiellement entre 45 et 84% en masse d'arylalcoxy-acrylate différent d'un 2-phénoxy-(2-éthoxy)ₙ-acrylate avec 4 ≤ n ≤ 6. De préférence, il en contient entre 70 et 80% en masse.

On ajoute au mélange un monomère à sous-chaine éthoxylée de type 2-phénoxy-(2-éthoxy)ₙ-acrylate, avec n égal à 4, 5 ou 6.

Le monomère à sous-chaine éthoxylée utilisé peut avantageusement être le 2-phénoxy-2-éthoxy-2-éthoxy-2-éthoxy-2-éthoxy-acrylate (n=4), également appelé 2-phénoxy-2-tétraéthylène glycol acrylate ou acrylate de 2-phénoxy-2-tétraéthylène glycol ou mono ester acrylique du phényléther du tétraéthylène glycol ou encore 4PEA.

Le mélange initial avant polymérisation comprend préférentiellement entre 3 et 15% en masse de 2-phénoxy-(2-éthoxy)ₙ-acrylate, plus préférentiellement entre 4 et 10%, et de préférence autour de 6%.

Ce monomère permet de faire baisser la température de transition vitreuse du polymère résultant tout en lui conférant une certaine hydrophilie.

Comme il sera démontré par la suite, il permet en outre, avantageusement, de faire baisser le caractère autocollant des surfaces sur elles-mêmes et de très fortement réduire la sensibilité au blanchiment du matériau polymère obtenu.

Pour diminuer le caractère collant du matériau à l'état humide, le polymère final doit avoir une quantité suffisante de fonctions hydroxyles en surface. L'eau forme ainsi un film continu à la surface du matériau qui empêche le matériau de coller sur lui-même.

On ajoute ainsi au mélange des monomères hydroxylés : un acrylate hydroxylé et un méthacrylate hydroxylé, qui augmentent la tension de surface et l'affinité de la surface avec l'eau du polymère résultant.

Ces polymères contribuent ainsi à éviter que le matériau, et donc la lentille fabriquée à partir de celui-ci, ne blanchisse au contact prolongé de l'eau à 35°C par migration d'espèces non liées au réseau polymère et ayant une forte affinité avec l'eau.

Pour être compatibles avec le cahier des charges ces monomères ne doivent pas présenter, à l'état polymérisé et sec, une température de transition vitreuse trop élevée, c'est-à-dire supérieure à 10°C.

L'acrylate hydroxylé utilisé est par exemple un mono-acrylate de dihydroxy-alkyle ou un mono-acrylate de dihydroxy-éthoxy-alkyle dont la chaîne alkyle du glycol comporte de 3 à 6 atomes de carbone. On peut citer par exemple l'acrylate de 4-hydroxy-butyle également appelé 4-hydroxy-butyl-acrylate ou acrylate de butanediol, l'acrylate d'hexanediol ou le monoacrylate de triéthylène glycol.

Le méthacrylate hydroxylé utilisé est préférentiellement un monométhacrylate de dihydroxy-alkyle ou un monométhacrylate de dihydroxy-éthoxy-alkyle dont la chaîne alkyle du glycol comporte de 3 à 6 atomes de carbone. Il s'agit par exemple du méthacrylate d'hydroxy-éthyle, du monométhacrylate de butanediol, du monométhacrylate d'hexanediol ou du monométhacrylate de triéthylène glycol, les glycols à chaîne de plus de 3 atomes de carbone étant préférés car ils présentent une température de transition vitreuse inférieure à celle du méthacrylate d'hydroxy-éthyle.

La proportion de ces monomères hydroxylés dans le mélange avant polymérisation ne doit cependant pas être trop importante pour que le matériau résultant reste globalement hydrophobe et n'absorbe pas plus de 5% d'eau à 35°C.

Avantageusement, l'acrylate hydroxylé et le méthacrylate hydroxylé représentent ensemble préférentiellement entre 10 et 20% en masse du mélange, et plus préférentiellement autour de 11 à 15% du mélange.

La proportion relative de ces deux monomères hydroxylés l'un par rapport à l'autre peut varier selon les cas de 20 à 80% pour l'un et inversement pour l'autre en fonction de la température de transition vitreuse souhaitée.

Le mélange contient également des composés réticulants permettant d'obtenir à la polymérisation un réseau macromoléculaire tridimensionnel et non des polymères linéaires. Pour obtenir un tel maillage, on ajoute des monomères difonctionnels : un diacrylate de diol ethoxylé et un diméthacrylate de diol éthoxylé.

Ces composés réticulants comportent des fonctions éthoxy de façon à ne pas augmenter la température de transition vitreuse du matériau final et simultanément à maintenir un niveau d'hydrophilie homogène avec le reste de la composition.

Le diacrylate de diol éthoxylé utilisé est préférentiellement le diacrylate de triéthylène glycol ou le diacrylate de tétraéthylène glycol.

Le diméthacrylate de diol éthoxylé utilisé peut être le diméthacrylate de triéthylène glycol ou le diméthacrylate de tétraéthylène glycol.

La quantité de réticulants doit être suffisante pour que, dans le polymère final, il ne reste pas trop de longues chaînes pendantes riches en arylalkoxy-acrylate qui augmentent le caractère collant du polymère.

En outre, plus le taux de réticulation est important et plus le temps de relaxation du polymère est court, la lentille se déployant plus vite dans l'oeil à température de transition vitreuse constante.

Mais d'autre part, le polymère résultant devient cassant lorsque son taux de réticulation est trop important.

La quantité de diacrylate de diol éthoxylé et de diméthacrylate de diol éthoxylé doit donc être soigneusement choisie. De préférence, on ajoute au mélange ces composés réticulants en quantités telles que le niveau de réticulation final soit une masse entre noeuds de réticulation comprise entre 2000 g/M et 10000 g/M.

Avantageusement, l'ensemble diacrylate de diol éthoxylé et diméthacrylate de diol éthoxylé représente préférentiellement entre 1 et 3% en masse du mélange, la proportion relative entre le diacrylate de diol éthoxylé et le diméthacrylate de diol éthoxylé variant de préférence de 20 à 80% de l'un par rapport à l'autre et inversement.

En résumant les considérations détaillées ci-dessus, on peut imaginer un mélange particulier de monomères conduisant par polymérisation radicalaire à un mode de réalisation préférentiel du matériau selon l'invention.

Ce mélange comprend préférentiellement au moins les monomères suivants : le 2-phénoxy-éthylacrylate ; le 2-phénoxy-2-éthoxy-2-éthoxy-2-éthoxy-2-éthoxy-acrylate ; le 4-hydroxy-butyl-acrylate ; le méthacrylate d'hydroxy-éthyle ; le diacrylate de tétraéthylène glycol ; et le diméthacrylate de tétraéthylène glycol.

Le matériau selon l'invention n'est cependant pas limité aux monomères précédemment cités, d'autres monomères pouvant bien entendu être rajoutés au mélange, comme par exemple le diméthacrylate de triéthylène glycol qui peut être ajouté en plus du diacrylate de tétraéthylène glycol et du diméthacrylate de tétraéthylène glycol afin d'ajuster le niveau de réticulation.

Il est également possible de rajouter parmi les monomères un ou plusieurs colorant(s) polymérisable(s) ou non polymérisable(s), ou un ou plusieurs agent(s) anti UV dont la fonction au sein du matériau final est d'absorber les rayonnements ultra-violets. Il peut s'agir par exemple du 2-[3-(2H-benzotriazol-2-yl)-4-hydroxyphenyl]ethyl-méthacrylate que l'on utilise préférentiellement en proportion comprise entre 0.1% et 1% en masse, et par exemple avec une teneur de 0,5%.

Tout autre monomère ou tout autre constituant polymérisable ou non polymérisable, imaginable par l'homme du métier, de fonction quelconque, pourra être rajouté dans le mélange sans sortir de la présente invention, tant que sa présence ne modifie pas les propriétés générales du matériau polymère résultant d'une façon qui le rende inadapté à la réalisation de lentilles intraoculaires.

Le matériau polymère acrylique selon l'invention est obtenu par un procédé de polymérisation radicalaire qui comprend une seule étape de polymérisation, la polymérisation et la réticulation ayant lieu simultanément au cours de la même étape du procédé.

Pour cela, on commence par réaliser un mélange de tous les monomères nécessaires à la réalisation du matériau polymère selon l'invention.

Ce mélange contient au moins un arylalcoxy-acrylate différent d'un 2-phénoxy-(2-éthoxy)ₙ-acrylate avec 4 ≤ n ≤ 6 , un 2-phénoxy-(2-éthoxy)n-acrylate avec 4 ≤ n ≤ 6, un acrylate hydroxylé, un méthacrylate hydroxylé, un diacrylate de diol éthoxylé, et un diméthacrylate de diol éthoxylé.

Il contient en outre au moins un agent de transfert. Il peut s'agir d'un produit halogéné ou plus préférentiellement d'un composé de la famille des thiols, tel que par exemple le butane thiol ou l'octane thiol.

On définira un agent de transfert comme étant un composé chimique qui lors de la polymérisation radicalaire capte un radical sur un macromère et le transfère sur un nouveau monomère pour propager la réaction.

Les agents de transfert sont des composés chimiques bien connus de l'homme du métier. On en trouve par exemple une définition et une liste complète dans l'ouvrage de référence suivant : « Polymer Handbook, fourth edition, Volume 1, Editors : J. Brandrup, E. H. Immergut, and E.A.Grulke » dans l'article intitulé « Transfer constants to monomers, polymers, catalysts and initiators, solvents and additives, and sulfur compounds in free radical polymerization » de A. Ueda et S. Nagai, qui se trouve partie II, page 97 et suivantes.

Les polymères acryliques fortement réticulés sont connus pour être fragiles et cassants, ce qui se traduit par un allongement à la rupture inversement proportionnel à leur taux de réticulation.

L'ajout d'une petite quantité d'agent de transfert au mélange initial de monomères, avant polymérisation et donc réticulation, permet de réduire cette propriété particulièrement gênante pour l'application visée, les lentilles intraoculaires subissant en effet une déformation et un étirement très importants lors de leur implantation dans l'oeil du patient.

L'agent de transfert permet avantageusement d'augmenter l'aptitude à la déformation sans rupture du matériau polymère résultant. L'ajout au mélange d'un agent de transfert permet d'avoir un taux de réticulation élevé, tout en gardant un allongement à la rupture important.

Cet agent de transfert arrête localement la polymérisation en transférant le radical d'un macromère réticulé à un monomère. La formation du maillage tridimensionnel est ainsi localement interrompue et l'on obtient à ce niveau une maille coupée avec une chaîne pendante courte reliée au réseau mais dont l'autre extrémité reste libre. L'agent de transfert permet ainsi d'obtenir un maillage plus lâche capable de s'étirer davantage sans rupture.

A la fin de la polymérisation en présence de l'agent de transfert, le copolymère réticulé obtenu se présente sous la forme d'un réseau macromoléculaire tridimensionnel à chaînes pendantes.

Avantageusement, une très petite quantité d'agent de transfert est nécessaire à l'obtention de ce résultat. Le mélange initial contient ainsi préférentiellement entre 0,03% et 0,2% en masse d'agent de transfert, plus préférentiellement encore entre 0,04% et 0,15% d'agent de transfert, 0,05% étant une valeur préférée pour le butane thiol et 0,1 % pour l'octane thiol.

En plus des monomères, le mélange initial peut contenir un certain nombre de composés supplémentaires de nature différente, par exemple nécessaires au bon déroulement de la réaction.

Il comprend par exemple un ou plusieurs composés initiateurs qui servent à amorcer la réaction de polymérisation en créant des sites actifs sur les monomères. Il(s) permet(tent) ainsi de régler la cinétique de la réaction de polymérisation.

Ce ou ces composés initiateurs peuvent par exemple être choisis parmi les peroxydes d'alkyl, le diperoxyde de lauroyl communément appelé peroxyde de lauroyl, le 1,1-di-ter-butylperoxycyclohexane ou le tert-amyl-peroxy-2-ethyl-hexyl-carbonate également appelé Taec.

Ce ou ces composés sont ajoutés au mélange en très petite quantité, le mélange comprenant par exemple entre 0,3 et 2% en masse de composé initiateur.

Afin de rendre ce descriptif plus complet, on va maintenant décrire un exemple de procédé d'obtention du matériau polymère acrylique selon l'invention à partir du mélange initial détaillé ci-dessus.

Pour réaliser la polymérisation recherchée, on commence par mélanger entre eux tous les différents monomères nécessaires à la réaction. Avantageusement, ces monomères sont solubles les uns dans les autres et une simple agitation suffit à réaliser un mélange homogène de ceux-ci.

On ajoute ensuite à ce mélange le composé ou les composés initiateurs nécessaires pour déclencher la réaction de polymérisation.

L'agent de transfert est également rajouté au mélange avant ou après le composé initiateur.

On réalise ensuite la polymérisation par voie radicalaire, en une seule étape.

Pour cela, de petites quantités de ce mélange sont placées dans des moules et chauffées par exemple à une température comprise entre 75°C et 95°C.

Une fois la réaction terminée et après refroidissement, on procède au démoulage du polymère.

Les moules sont préférentiellement choisis de façon à obtenir après démoulage des blocs de polymère de forme générale cylindrique de faible hauteur, de type «jeton» ou «palet». Une telle forme est parfaitement adaptée à un usinage ultérieur de ces blocs de polymère en vue d'obtenir les lentilles intraoculaires.

Bien entendu, un moulage direct des lentilles intraoculaires est également possible avec un moule adapté.

Les blocs de polymère sont ensuite purifiés, afin de les débarrasser des monomères n'ayant pas réagi et des produits résiduels provenant notamment de la synthèse de chacun des monomères utilisés.

Les blocs de matériau polymère sont alors prêts à être usinés, à une température inférieure à la température de transition vitreuse du polymère, pour réaliser les lentilles intraoculaires selon l'invention.

Afin de parfaitement décrire l'invention, deux exemples de matériau polymère acrylique selon l'invention sont détaillés ci-dessous.

### Exemple 1 :

Le matériau polymère acrylique a été obtenu par polymérisation radicalaire à partir du mélange initial suivant : (Les quantités sont exprimées en pourcentages massiques du mélange initial avant polymérisation.)

### MONOMÈRES :

- 2 phénoxy-éthylacrylate 76,1%
- 2-phénoxy-tétraéthylène glycol acrylate 6%
- acrylate de 4-hydroxy-butyle 9,7%
- méthacrylate d'hydroxy-éthyle 4%
- diacrylate de tétraéthylène glycol 0,6%
- diméthacrylate de tétraéthylène glycol 2%
- agent anti-UV 0,5%

### COMPOSÉ INITIATEUR :

- diperoxyde de lauroyle 0,5%
- tert-amyl-peroxy-2-ethyl-hexyl-carbonate 0,5%

### AGENT DE TRANSFERT :

- octane thiol 0,1%

On obtient ainsi, après polymérisation à 90°C pendant une durée de 10 heures, un matériau polymère acrylique présentant un indice optique égal à 1,545 et une température de transition vitreuse sensiblement égale à 10°C.

### Exemple 2 :

Le matériau polymère acrylique a été obtenu par polymérisation radicalaire à partir du mélange initial suivant : (Les quantités sont exprimées en pourcentages massiques du mélange initial avant polymérisation.)

### MONOMÈRES :

- 2 phénoxy-éthylacrylate 77%
- 2-phénoxy-tétraéthylène glycol acrylate 8%
- acrylate de 4-hydroxy-butyle 8%
- méthacrylate d'hydroxy-éthyle 3%
- diacrylate de tétraéthylène glycol 0,4%
- diméthacrylate de tétraéthylène glycol 1,5%
- diméthacrylate de triéthylène glycol 0,5%
- agent anti-UV 0,5%

### COMPOSÉ INITIATEUR :

- diperoxyde de lauroyle 1%

### AGENT DE TRANSFERT :

- octane thiol 0,1%

On obtient ainsi, après polymérisation à 90°C pendant une durée de 10 heures, un matériau polymère acrylique présentant un indice optique égal à 1,545 et une température de transition vitreuse sensiblement égale à 9°C.

Pour mettre en évidence les avantages surprenants du matériau selon l'invention par rapport à ceux de l'art antérieur et démontrer sa faible sensibilité au glistening et son caractère peu collant sur lui-même, une série de tests a été réalisée afin de comparer les propriétés du matériau selon la présente invention par rapport à celui décrit dans la demande de brevet antérieure FR 2 930 731.

Plusieurs matériaux polymère ont été fabriqués à partir du même arylalcoxy-acrylate (le 2-phénoxy-éthylacrylate), acrylate hydroxylé (l'acrylate de 4-hydroxy-butyle), méthacrylate hydroxylé (le méthacrylate d'hydroxy-éthyle), diacrylate de diol (le diacrylate de tétraéthylène glycol) et diméthacrylate de diol (le diméthacrylate de tétraéthylène glycol).

Certains d'entre eux ont été réalisés selon la formule de la demande de brevet antérieure FR 2 930 731, en y ajoutant un alkylacrylate, à savoir l'acrylate de butyle dit ABU.

Les autres ont été réalisés selon la formule de la présente invention, en y ajoutant à la place et dans les mêmes quantités un 2-phénoxy-(2-éthoxy)n-acrylate, avec 4 ≤ n ≤ 6, à savoir le 2-phénoxy-2-éthoxy-2-éthoxy-2-éthoxy-2-éthoxy-acrylate dit 4PEA.

Une même quantité d'agent de transfert (octane thiol) a été ajoutée dans tous les cas.

Pour obtenir différents matériaux polymère à tester, on a fait varier le pourcentage des monomères hydroxylés (acrylate et méthacrylate hydroxylé) et celui de l'acrylate de butyle (ABU) pour les polymères selon l'art antérieur, et de la même façon le pourcentage des monomères hydroxylés (acrylate et méthacrylate hydroxylé) et celui de la 4PEA pour les polymères selon l'invention, les autres monomères restant en quantités identiques.

On a ensuite soumis les matériaux polymère obtenus à deux séries de test : un test de mesure du glistening et un test d'auto-adhésion dans l'eau.

### Test de mesure du glistening :

On a réalisé des lentilles intraoculaires avec les différents matériaux polymère obtenus et on les a plongées dans de l'eau à 35°C pendant un mois.

On leur fait alors passer un test de mesure de l'indice de glistening qui a été mis au point par L. Werner et qui consiste à observer chaque lentille au microscope, à réaliser une photographie à la lampe à fente, à compter le nombre de points de glistening visibles sur cette photographie et à le comparer à une échelle de référence afin d' attribuer à la lentille un indice compris entre 0 et 5.

Les résultats obtenus par les différents matériaux testés sont rassemblés dans le tableau ci-dessous :

| **Polymère de l'art antérieur (selon** FR 2 930 731**)** | | | **Polymère selon l'invention** | | |
|---|---|---|---|---|---|
| Pourcentage de monomères hydroxylés (acrylate + méthacrylate) | Pourcentage d'ABU | **Indice de glistening** | Pourcentage de monomères hydroxylés (acrylate + méthacrylate) | Pourcentage de 4PEA | **Indice de glistening** |
| 13 | 6 | **2** | 13 | 6 | **0,5** |
| 10 | 6 | **3** | 10 | 6 | **0,5** |
| 15 | 4 | **2** | 15 | 4 | **1,5** |
| 13 | 10 | **5** | 13 | 10 | **0,5** |
| 15 | 8 | **4** | 15 | 8 | **2** |

On constate qu'avec un pourcentage de monomères hydroxylés identique et un pourcentage de 4PEA comparable à celui de l'acrylate de butyl (ABU), les matériaux polymère selon l'invention présentent un indice de glistening nettement inférieur à celui des matériaux polymère antérieurs équivalents.

### Test d'auto-adhésion dans l'eau :

A partir des matériaux polymère obtenus, on a réalisé des bandes de 5 cm de long, 3 cm de large et 3 mm d'épaisseur.

Ces bandes ont été placées dans de l'eau à 25°C. Elles ont ensuite été pliées au niveau de leur zone centrale et repliées sur elles-mêmes de manière à faire adhérer les surfaces intérieures qui se retrouvent en contact. Après avoir maintenu une pression en appuyant parallèlement au niveau de la pliure pendant une minute, les bandes repliées sont libérées de toute contrainte.

On mesure alors le temps nécessaire à ces bandes pour se déployer complètement et retrouver une configuration horizontale. Ce temps, mesuré en seconde, est caractéristique de la pégosité du matériau (en anglais « tack ») c'est-à-dire de son caractère collant sur lui-même.

Les résultats obtenus par les différents matériaux testés ont été rassemblés dans le tableau ci-dessous :

| **Polymère de l'art antérieur (selon** FR 2 930 731**)** | | | **Polymère selon l'invention** | | |
|---|---|---|---|---|---|
| Pourcentage de monomères hydroxylés (acrylate + méthacrylate) | Pourcentage d'ABU | **Temps d'adhésion (secondes)** | Pourcentage de monomères hydroxylés (acrylate + méthacrylate) | Pourcentage de 4PEA | **Temps d'adhésion (secondes)** |
| 13 | 6 | **30** | 13 | 6 | **10** |
| 10 | 6 | **30** | 10 | 6 | **15** |
| 15 | 4 | **35** | 15 | 4 | **10** |
| 13 | 10 | **> 60** | 13 | 10 | **20** |
| 15 | 8 | **20** | 15 | 8 | **5** |

On constate qu'avec un pourcentage de monomères hydroxylés identique et un pourcentage de 4PEA comparable à celui de l'acrylate de butyl (ABU), les matériaux polymère selon l'invention mettent beaucoup moins de temps à se déployer que les matériaux polymère antérieurs équivalents. Ils sont donc beaucoup moins collants sur eux-mêmes que les matériaux polymère antérieurs ce qui est un avantage considérable lors de la pose des lentilles intraoculaires.

## Revendications

1. Matériau polymère acrylique, hydrophobe, destiné à la réalisation de lentilles intraoculaires, **caractérisé en ce qu'**il s'agit d'un copolymère réticulé d'au moins les monomères suivants :
- un arylalcoxy-acrylate différent d'un 2-phénoxy-(2-éthoxy)ₙ-acrylate avec 4 ≤ n ≤ 6;
- un 2-phénoxy-(2-éthoxy)ₙ-acrylate avec 4 ≤ n ≤ 6;
- un acrylate hydroxylé ;
- un méthacrylate hydroxylé ;
- un diacrylate de diol éthoxylé ; et
- un diméthacrylate de diol éthoxylé,
et **en ce que** ledit copolymère réticulé se présente sous la forme d'un réseau macromoléculaire tridimensionnel à chaînes pendantes, du fait de la présence d'au moins un agent de transfert dans le mélange de monomères lors de la réticulation.

2. Matériau polymère acrylique selon la revendication précédente **caractérisé en ce que** l'arylalcoxy-acrylate différent d'un 2-phénoxy-(2-éthoxy)ₙ-acrylate avec 4 ≤ n ≤ 6 est un composé choisi parmi le 2-phénoxy-éthylacrylate, le 2-phénoxy-2-éthoxy-éthylacrylate ou le 2-phénoxy-2-éthoxy-2-éthoxy-éthylacrylate.

3. Matériau polymère acrylique selon l'une quelconque des revendications précédentes **caractérisé en ce que** le 2-phénoxy-(2-éthoxy)ₙ-acrylate avec 4 ≤ n ≤ 6 est le 2-phénoxy-2-éthoxy-2-éthoxy-2-éthoxy-2-éthoxy-acrylate.

4. Matériau polymère acrylique selon l'une quelconque des revendications précédentes **caractérisé en ce que** l'acrylate hydroxylé est un monoacrylate de dihydroxy-alkyle ou un monoacrylate de dihydroxy-éthoxy-alkyle dont la chaîne alkyle du glycol comporte de 3 à 6 atomes de carbone ; et **en ce que** le méthacrylate hydroxylé est un monométhacrylate de dihydroxy-alkyle ou un monométhacrylate de dihydroxy-éthoxy-alkyle dont la chaîne alkyle du glycol comporte de 3 à 6 atomes de carbone.

5. Matériau polymère acrylique selon l'une quelconque des revendications précédentes **caractérisé en ce que** le diacrylate de diol ethoxylé est le diacrylate de triéthylène glycol ou le diacrylate de tétraéthylène glycol ; et **en ce que** le diméthacrylate de diol éthoxylé est le diméthacrylate de triéthylène glycol ou le diméthacrylate de tétraéthylène glycol.

6. Matériau polymère acrylique selon l'une quelconque des revendications précédentes **caractérisé en ce qu'**il s'agit d'un copolymère réticulé d'au moins les monomères suivants :
- le 2-phénoxy-éthylacrylate ;
- le 2-phénoxy-2-éthoxy-2-éthoxy-2-éthoxy-2-éthoxy-acrylate ;
- l'acrylate de 4-hydroxy-butyle ;
- le méthacrylate d'hydroxy-éthyle ;
- le diacrylate de tétraéthylène glycol ;
- le diméthacrylate de tétraéthylène glycol.

7. Matériau polymère acrylique selon la revendication 1 **caractérisé en ce qu'**il s'agit d'un copolymère réticulé d'au moins les monomères selon la revendication 1 dans les proportions massiques suivantes :
- entre 45 et 84% d'arylalcoxy-acrylate différent d'un 2-phénoxy-(2-éthoxy)ₙ-acrylate avec 4 ≤ n ≤ 6
- entre 3 et 15% de 2-phénoxy-(2-éthoxy)ₙ-acrylate, avec 4 ≤ n ≤ 6 ;
- entre 11 et 15% d'acrylate hydroxylé et de méthacrylate hydroxylé ;
- entre 1 et 3% de diacrylate de diol éthoxylé et de diméthacrylate de diol éthoxylé.

8. Matériau polymère acrylique selon la revendication 7 **caractérisé en ce que** la proportion relative entre l'acrylate hydroxylé et le méthacrylate hydroxylé et entre le diacrylate de diol éthoxylé et le diméthacrylate de diol éthoxylé varie pour chaque couple de 20 à 80% de l'un par rapport à l'autre.

9. Matériau polymère acrylique selon la revendication 7 **caractérisé en ce que** le 2-phénoxy-(2-éthoxy)ₙ-acrylate est le 2-phénoxy-2-éthoxy-2-éthoxy-2-éthoxy-2-éthoxy-acrylate est **en ce que** la proportion massique du 2-phénoxy-2-éthoxy-2-éthoxy-2-éthoxy-2-éthoxy-acrylate est comprise entre 4 et 10%.

10. Matériau polymère acrylique selon l'une quelconque des revendications précédentes **caractérisé en ce qu'**il s'agit d'un copolymère réticulé d'au moins les monomères selon la revendication 1 et d'un monomère absorbeur d'UV.

11. Procédé de fabrication d'un matériau polymère acrylique selon l'une quelconque des revendications 1 à 10 **caractérisé en ce que** :
• on réalise un mélange contenant au moins :
- un arylalcoxy-acrylate différent d'un 2-phénoxy-(2-éthoxy)ₙ-acrylate avec 4≤n≤6;
- un 2-phénoxy-(2-éthoxy)ₙ-acrylate avec 4 ≤ n ≤ 6;
- un acrylate hydroxylé ;
- un méthacrylate hydroxylé ;
- un diacrylate de diol éthoxylé ;
- un diméthacrylate de diol éthoxylé, et
- un agent de transfert ;
• on polymérise ce mélange par voie radicalaire, en une seule étape de polymérisation, de manière à obtenir par cette polymérisation un réseau macromoléculaire tridimensionnel à chaînes pendantes.

12. Procédé de fabrication d'un matériau polymère acrylique selon la revendication 11 **caractérisé en ce que** le mélange comprend en outre au moins un composé initiateur.

13. Procédé de fabrication selon la revendication 12 **caractérisé en ce que** l'au moins un composé initiateur est choisi parmi les peroxydes d'alkyl, le diperoxyde de lauroyl, le 1,1-di-ter-butylperoxycyclohexane et le tert-amyl-peroxy-2-ethyl-hexyl-carbonate.

14. Procédé de fabrication selon l'une quelconque des revendications 11 à 13 **caractérisé en ce que** l'agent de transfert est le butane thiol ou l'octane thiol.

15. Procédé de fabrication selon l'une quelconque des revendications 11 à 14 **caractérisé en ce que** le mélange comprend entre 0,03 et 0,2% en masse d'agent de transfert.

16. Lentille intraoculaire à implanter chirurgicalement dans le sac cristallinien d'un patient, en remplacement de son cristallin naturel, **caractérisé en ce qu'**elle est réalisée à partir d'un matériau polymère acrylique selon l'une quelconque des revendications 1 à 10.

## Patentansprüche

1. Hydrophobes Acryl-Polymermaterial zur Herstellung von Intraokular-Linsen, **dadurch gekennzeichnet, dass** es sich um ein vernetztes Copolymer aus mindestens den folgenden Monomeren handelt:
- Arylalcoxy-Acrylat, verschieden von einem 2-Phenoxy-(2-ethoxy)ₙ -Acrylat mit 4 ≤ n ≤ 6;
- 2-Phenoxy-(2-ethoxy)ₙ -Acrylat mit 4 ≤ n ≤ 6;
- hydroxyliertes Acrylat;
- hydroxyliertes Methacrylat;
- Diacrylat von ethoxyliertem Diol; und
- Dimethacrylat von ethoxyliertem Diol,
und dadurch dass das genannte vernetzte Copolymer die Form eines dreidimensionalen, makromolekularen Netzes mit Seitenketten hat, aufgrund des Vorhandenseins von mindestens einem Transfermittel in der Monomer - Mischung bei der Vernetzung.

2. Acryl-Polymermaterial gemäß dem vorhergehenden Anspruch, **dadurch gekennzeichnet, dass** das Arylalcoxy-Acrylat, verschieden von einem 2-Phenoxy-(2-ethoxy)ₙ -Acrylat mit 4 ≤ n ≤ 6, eine Verbindung ist, die aus 2-Phenoxy-Ethylacrylat, 2-Phenoxy-2-ethoxy-Ethylacrylat oder 2-Phenoxy-2-ethoxy-2-ethoxy-Ethylacrylat ausgewählt wird.

3. Acryl-Polymermaterial gemäß einem der vorhergehenden Ansprüche, **dadurch gekennzeichnet, dass** es sich bei dem 2-Phenoxy-(2-ethoxy)ₙ -Acrylat mit 4 ≤ n ≤ 6 um 2-Phenoxy-2-ethoxy-2-ethoxy-2-ethoxy-2-ethoxy-Acrylat handelt.

4. Acryl-Polymermaterial gemäß einem der vorhergehenden Ansprüche, **dadurch gekennzeichnet, dass** das hydroxylierte Acrylat ein Dihydroxy-Alkyl-Monoacrylat oder ein Dihydroxy-ethoxy-Alkyl-Monoacrylat ist, bei dem die Alkylkette des Glykols 3 bis 6 Kohlenstoffatome enthält; und dadurch dass das hydroxylierte Methacrylat ein Dihydroxy-Alkyl-Monomethacrylat oder ein Dihidroxy-ethoxy-Alkyl-Monomethacrylat ist, bei dem die Alkylkette des Glykols 3 bis 6 Kohlenstoffatome enthält.

5. Acryl-Polymermaterial gemäß einem der vorhergehenden Ansprüche, **dadurch gekennzeichnet, dass** es sich bei dem ethoxylierten Diol-Diacrylat um Triethylenglykol-Diacrylat oder Tetraethylenglykol-Diacrylat handelt; und dass es sich bei dem ethoxylierten Diol-Dimethacrylat um Triethylenglykol-Dimethacrylat toder Tetraethylenglykol - Dimethacrylat handelt.

6. Acryl-Polymermaterial gemäß einem der vorhergehenden Ansprüche, **dadurch gekennzeichnet, dass** es sich um ein vernetztes Copolymer mit mindestens den folgenden Monomeren handelt:
- 2-Phenoxy-Ethylacrylat ;
- 2-Phenoxy-2-ethoxy-2-ethoxy-2-ethoxy-2-ethoxy-Acrylat ;
- 4-hydroxy-butyl-Acrylat;
- Hydroxy-ethyl-Methacrylat;
- Tetraethylen-Glycol-Diacrylat;
- Tetraethylen-Glycol-Dimethacrylat.

7. Acryl-Polymermaterial gemäß Anspruch 1, **dadurch gekennzeichnet, dass** es sich um ein vernetztes Copolymer mit mindestens den Monomeren gemäß Anspruch 1 im folgenden Masseanteilen handelt:
- zwischen 45 und 84% Arylalcoxy - Acrylat, verschieden von einem 2-Phenoxy-(2-ethoxy)ₙ-Acrylat mit 4 ≤ n ≤ 6
- zwischen 3 und 15 % 2-Phenoxy-(2-ethoxy)ₙ-Acrylat mit 4 ≤ n ≤ 6;
- zwischen 11 und 15 % hydroxyliertes Acrylat und hydroxyliertes Methacrylat;
- zwischen 1 und 3 % ethoxyliertes Diol-Diacrylat und ethoxyliertes Diol-Dimethacrylat

8. Acryl-Polymermaterial gemäß Anspruch 7, **dadurch gekennzeichnet, dass** das relative Verhältnis zwischen hydroxyliertem Acrylate und hydroxyliertem Methacrylat und zwischen dem ethoxylierten Diol-Diacrylat und ethoxyliertem Diol-Dimethacrylat für jedes Paar zwischen 20 und 80 % des einen, bezogen auf das andere, variiert.

9. Acryl-Polymermaterial gemäß Anspruch 7, **dadurch gekennzeichnet, dass** es sich bei dem 2-Phenoxy-(2-ethoxy)ₙ-Acrylat um 2-Phenoxy-2 -ethoxy-2-ethoxy-2-ethoxy-2-ethoxy-Acrylat handelt, dadurch dass das Masseverhältnis von 2-Phenoxy-2-ethoxy-2-ethoxy-2-ethoxy-2-ethoxy-Acrylat zwischen 4 und 10% liegt.

10. Acryl-Polymermaterial gemäß einem der vorhergehenden Ansprüche, **dadurch gekennzeichnet, dass** es sich um ein vernetztes Copolymer mit mindestens den Monomeren gemäß Anspruch 1 und einem UV-absorbierenden Monomer handelt.

11. Verfahren zur Herstellung eines Acryl-Polymermaterials gemäß einem der Ansprüche 1 bis 10, **gekennzeichnet dadurch, dass**:
eine Mischung hergestellt wird, die mindestens enthält:
- ein Arylalcoxy-Acrylat verschieden von einem 2-Phenoxy-(2-ethoxy)ₙ-Acrylat mit 4 ≤ n ≤ 6;
- ein 2-Phenoxy-(2-ethoxy)ₙ-Acrylat mit 4 ≤ n ≤ 6;
- ein hydroxyliertes Acrylat;
- ein hydroxyliertes Methacrylat;
- ein Diacrylat von ethoxyliertem Diol; und
- ein Dimethacrylat von ethoxyliertem Diol, und
- ein Transfermittel
diese Mischung wird radikalreaktiv polymerisiert, in einem einzigen Polymerisationsschritt, so dass sich durch diese Polymerisation ein dreidimensionales makromolekulares Netz mit Seitenketten ergibt.

12. Verfahren zur Herstellung eines Acryl-Polymermaterials gemäß Anspruch 11, **dadurch gekennzeichnet, dass** die Mischung außerdem mindestens eine Initiatorverbindung enthält.

13. Herstellungsverfahren gemäß Anspruch 12, **dadurch gekennzeichnet, dass** die mindestens eine Initiatorverbindung aus Alkylperoxyde, Lauroyl-diperoxyd, 1,1-Di-ter-butylperoxycyclohexan und Tert-amyl-peroxy-2-ethyl-hexyl-carbonat ausgewählt wird.

14. Herstellungsverfahren gemäß einem der Ansprüche 11 bis 13, **dadurch gekennzeichnet, dass** es sich bei dem Transfermittel um Butanthiol oder Oktanthiol handelt.

15. Herstellungsverfahren gemäß einem der Ansprüche 11 bis 14, **dadurch gekennzeichnet, dass** die Mischung zwischen 0,03 und 0,2 Masse - % Transfermittel enthält.

16. Intraokular-Linse zur chirurgischen Implantation in die Linsenkapsel eines Patienten als Ersatz für die natürliche Linse, **dadurch gekennzeichnet, dass** sie aus einem Acryl-Polymermaterial gemäß einem der Ansprüche 1 bis 10 hergestellt wird.

## Claims

1. Hydrophobic acrylic polymer material intended for the production of intraocular lenses, **characterized in that** it is a cross-linked co-polymer of at least the following monomers:
- an arylalcoxy-acrylate different from a 2-phenoxy-(2-ethoxy)ₙ-acrylate with 4 ≤ n ≤ 6;
- a 2-phenoxy-(2-ethoxy)ₙ-acrylate with 4 ≤ n ≤ 6;
- a hydroxylated acrylate;
- a hydroxylated methacrylate;
- an ethoxylated diol diacrylate; and
- an ethoxylated diol dimethacrylate;
and **in that** said cross-linked copolymer takes the form of a three-dimensional macromolecular network with hanging chains, due to the presence of at least one transfer agent in the monomer mixture during the cross-linking.

2. Acrylic polymer material according to the preceding claim, **characterized in that** the arylalcoxy-acrylate different from a 2-phenoxy-(2-ethoxy)ₙ acrylate with 4 ≤ n ≤ 6 is a compound selected from 2-phenoxy-ethylacrylate, 2-phenoxy-2-ethoxy-ethylacrylate or 2-phenoxy-2-ethoxy-2-ethoxy-ethylacrylate.

3. Acrylic polymer material according to any one of the preceding claims, **characterized in that** the 2-phenoxy-(2-ethoxy)ₙ-acrylate with 4 ≤ n ≤ 6 is 2-phenoxy-2-ethoxy-2-ethoxy-2-ethoxy-2-ethoxy-acrylate.

4. Acrylic polymer material according to any one of the preceding claims, **characterized in that** the hydroxylated acrylate is a dihydroxy-alkyl monoacrylate or a dihydroxy-ethoxy-alkyl monoacrylate of which the alkyl chain of the glycol contains 3 to 6 carbon atoms, and **in that** the hydroxylated methacrylate is a dihydroxy-alkyl monomethacrylate or a dihydroxy-ethoxy-alkyl monomethacrylate of which the alkyl chain of the glycol contains 3 to 6 carbon atoms.

5. Acrylic polymer material according to any one of the preceding claims, **characterized in that** the ethoxylated diol diacrylate is triethylene glycol diacrylate or tetraethylene glycol diacrylate; and **in that** the ethoxylated diol dimethacrylate is triethylene glycol dimethacrylate or tetraethylene glycol dimethacrylate.

6. Acrylic polymer material according to any of the preceding claims, **characterized in that** it is a cross-linked co-polymer of at least the following monomers:
- 2-phenoxy-ethylacrylate;
- 2-phenoxy-2-ethoxy-2-ethoxy-2-ethoxy-2-ethoxy-acrylate;
- 4-hydroxy-butyl acrylate;
- hydroxy-ethyl methacrylate;
- tetraethylene glycol diacrylate;
- tetraethylene glycol dimethacrylate.

7. Acrylic polymer material according to claim 1, **characterized in that** it is a cross-linked co-polymer of at least the monomers according to claim 1, in the following weight proportions:
- between 45 and 84% of arylalcoxy-acrylate different from a 2-phenoxy-(2-ethoxy)ₙ-acrylate with 4 ≤ n ≤ 6;
- between 3 and 15% of 2-phenoxy-(2-ethoxy)ₙ-acrylate with 4 ≤ n ≤ 6;
- between 11 and 15% of hydroxylated acrylate and hydroxylated methacrylate;
- between 1 and 3% of ethoxylated diol diacrylate and ethoxylated diol dimethacrylate.

8. Acrylic polymer material according to claim 7, **characterized in that** the relative proportion between the hydroxylated acrylate and the hydroxylated methacrylate and between the ethoxylated diol diacrylate and the ethoxylated diol dimethacrylate varies for each pair from 20 to 80% of one relative to the other.

9. Acrylic polymer material according to claim 7, **characterized in that** the 2-phenoxy-(2-ethoxy)ₙ₋acrylate is 2-phenoxy-2-ethoxy-2-ethoxy-2-ethoxy-2-ethoxy-acrylate, and **in that** the weight proportion of 2-phenoxy-2-ethoxy-2-ethoxy-2-ethoxy-2-ethoxy-acrylate is comprised between 4 and 10%.

10. Acrylic polymer material according to any one of the preceding claims, **characterized in that** it is a cross-linked co-polymer of at least the monomers according to claim 1 and of a UV-absorbing monomer.

11. Method for the production of an acrylic polymer material according to any one of claims 1 to 10, **characterized in that**:
• a mixture is realized containing at least:
- an arylalcoxy-acrylate different from a 2-phenoxy-(2-ethoxy)ₙ-acrylate with 4 ≤ n ≤ 6;
- a 2-phenoxy-(2-ethoxy)ₙ-acrylate with 4 ≤ n ≤ 6;
- a hydroxylated acrylate;
- a hydroxylated methacrylate;
- an ethoxylated diol diacrylate;
- an ethoxylated diol dimethacrylate; and
- a transfer agent;
• this mixture is polymerized radically, in one single polymerization step, such that to obtain by this polymerization a three-dimensional macromolecular network with hanging chains.

12. Method for the production of an acrylic polymer material according to claim 11, **characterized in that** the mixture also includes at least one initiator compound.

13. Production method according to claim 12, **characterized in that** said at least one initiator compound is chosen from alkyl peroxides, lauroyl diperoxide, 1,1-di-ter-butylperoxycyclohexane, and tert-amyl-peroxy-2-ethyl-hexyl-carbonate.

14. Production method according to any one of claims 11 to 13, **characterized in that** the transfer agent is thiol butane or thiol octane.

15. Production method according to any one of claims 11 to 14, **characterized in that** the mixture comprises between 0.03 and 0.2% by weight of transfer agent.

16. Intraocular lens to be surgically implanted in the crystalline bag of a patient, as a replacement for his/her natural crystalline lens, **characterized in that** it is produced from an acrylic polymer material according to any one of claims 1 to 10.
